(19) Europäisches Patentamt
European Patent Office
Office européen des brevets

(11) **EP 4 607 528 A1**

(12) **EUROPEAN PATENT APPLICATION**

(43) Date of publication:
**27.08.2025   Bulletin 2025/35**

(21) Application number: **24158895.3**

(22) Date of filing: **21.02.2024**

(51) International Patent Classification (IPC):
**G16H 30/20** (2018.01)          **G16H 30/40** (2018.01)
**G16H 40/63** (2018.01)

(52) Cooperative Patent Classification (CPC):
**G16H 30/20; G16H 30/40; G16H 40/63**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
• **Arroyo Camejo, Silvia Bettina**
  **90763 Fürth (DE)**
• **Kapoor, Ankur**
  **Plainsboro, 08536 (US)**
• **Kühn, Bernd**
  **91080 Uttenreuth (DE)**
• **Tamersoy, Birgi**
  **91052 Erlangen (DE)**
• **Wohlers, Julian**
  **91052 Erlangen (DE)**

(74) Representative: **Siemens Healthineers**
**Patent Attorneys**
**Postfach 22 16 34**
**80506 München (DE)**

(54) **METHOD FOR MONITORING AND/OR CONTROLLING A MEDICAL IMAGING PROCEDURE AND METHODS FOR PROVIDING TRAINED MACHINE LEARNING MODELS**

(57)      Computer-implemented method for monitoring and/or controlling a medical imaging procedure on a patient (22), comprising the steps of:
- receiving a breathing information (24) concerning a breathing pattern of the patient (22),
- selecting a compliance class (25) from at least two possible compliance classes based on the breathing information (24), wherein at least one of the possible compliance classes corresponds to a compliance of the acquired breathing information (24) with a given desired breathing and/or breath-holding pattern (26), and
- on the one hand controlling the medical imaging procedure depending on the selected compliance class (25) and/or
- on the other hand outputting a non-compliance information (27) to a user and/or storing a non-compliance information (27) with an acquired medical image data (29), when the selected compliance class (25) does not indicate a compliance of the acquired breathing information (24) with the given desired breathing and/or breath-holding pattern (26).

FIG 2

**Description**

The invention concerns a computer-implemented method for monitoring and/or controlling a medical imaging procedure on a patient, methods for providing trained machine learning models, a data processing system, a computer program, and a computer-readable medium.

For many medical imaging protocols, in particular for many magnetic resonance imaging techniques, acquisitions require the patient to follow a particular breathing and/or breath-holding pattern. The compliance of the patient to a target breathing pattern is important for reducing motion artifacts and ensuring a good image quality, since the corresponding sequences are typically designed with this assumption.

In many medical imaging protocols, medical personnel overseeing medical imaging needs to recognize, whether the patient is following a desired breathing and/or breath-holding pattern, e.g., given instructions, while the imaging protocol is running. A sufficiently close monitoring can be challenging, e.g., in many x-ray and magnetic resonance imaging protocols.

In many cases motion artifacts or other issues caused by a patient not following the desired breathing and/or breath-holding pattern will only be detected when analyzing the final image data. This can lead to an extended time requirement for such imaging protocols to allow sufficient time for a repeat of the imaging sequence when it should be necessary and therefore to a lower utilization of the used imaging device or it might even require the patient to come back to an imaging facility for a further imaging after he has already left the facility.

The invention is therefore based on the problem of providing an improved way of monitoring and/or controlling a medical imaging procedure with respect to the compliance of the patient to a desired breathing and/or breath-holding pattern.

The problem is solved by a computer-implemented method for monitoring and/or controlling a medical imaging procedure on a patient, comprising the steps of:

- receiving a breathing information concerning a breathing pattern of the patient,
- selecting a compliance class from at least two possible compliance classes based on the breathing information, wherein at least one of the possible compliance classes corresponds to a compliance of the acquired breathing information with a given desired breathing and/or breath-holding pattern, and
- on the one hand controlling the medical imaging procedure depending on the selected compliance class and/or
- on the other hand outputting a non-compliance information to a user and/or storing a non-compliance information with an acquired medical image data, when the selected compliance class does not indicate a compliance of the acquired breathing information with the given desired breathing and/or breath-holding pattern.

As discussed in more detail below, various ways to detect a breathing state of the patient are already known and, e.g., used for gating purposes, e.g., to only take measurement data from a specific phase of the breathing cycle into account. While a medical expert could in principle, manually evaluate such a breathing information to determine, whether the patient complies to the given desired breathing and/or breath-holding pattern, this approach would be quite time-consuming and therefore would only allow for a determination, whether the desired breathing and/or breath-holding pattern was matched, after the imaging procedure is already finished and potentially after the patient has already left the imaging facility.

By the proposed automatic selection of a compliance class based on the breathing information, it is however possible, to already determine, whether the desired breathing and/or breath-holding pattern was matched sufficiently closely to likely provide a satisfactory imaging quality, while the imaging sequence is still running or even before the imaging sequence has started. This can allow for an adjustment of the parametrization of the imaging sequence on the fly, before it is even started or even while it is running, and/or for a quick abortion and restart of the imaging sequence, when it is already running. Overall, the required time and burden on the patient can be noticeably reduced by using the computer implemented method according to the present invention.

The desired breathing and/or breath-holding pattern can be specified by instructions that can be provided to the patient by medical personnel or, in a preferred embodiment, automatically by the medical imaging device.

In the simplest case, only two possible compliance classes can be used, wherein one of these compliance classes indicates a sufficient compliance of the patient, such that the medical imaging procedure can be performed without changes, and the other compliance class indicates, that the compliance is insufficient. When the compliance is insufficient, this can indicate, that a planned imaging sequence should not be started and/or should be modified and/or that an imaging sequence that is already running or finished should be repeated.

It is however also possible, to allow the selection between more than two possible compliance classes. This can, e.g., allow for multiple levels of compliance that can be classified, wherein the compliance can, e.g., be classified on a scale from 1 to 5. This can, e.g., be useful, when at least some of the compliance classes still allow for the imaging to proceed but can, e.g., require a modification of the imaging sequence and/or the postprocessing. It can therefore be advantageous, when the medical imaging procedure is controlled depending on the selected compliance class, even when the selected compliance class does indicate a sufficient compliance of the acquired breathing information with the given desired breathing and/or breath-holding pattern to allow for an imaging.

The breathing information can be based on the breathing of the patient during the medical imaging procedure and/or during a preparatory time interval before the imaging has started and/or during a further time interval after the imaging procedure has already ended. The acquisition of the breathing information can be performed before the beginning of the claimed computer-implemented method or it can be acquired as part of the step of the receiving of the breathing information.

The breathing information can be based on sensor data of at least one dedicated sensor. The dedicated sensor can, e.g., be a chest band measuring an extension of the chest due to breathing, a nasal airflow sensor, an optical camera, that can in particular detect optical fiducial markers, and/or a laser range sensor. Additionally or alternatively, the breathing information can be based on measurement data acquired by the medical imaging device used during the medical imaging procedure. It is, e.g., possible, that the medical imaging procedure is or comprises a magnetic resonance imaging. In this case a so-called pilot tone can be used to acquire measurement data concerning the movement of the diaphragm of the patient that corresponds to the breathing motion of the patient and can therefore be used as the breathing information.

The breathing information can, in particular, describe a chronological sequence of multiple measurements concerning a respective breathing status, wherein the selection of the selected compliance class depends on the chronological sequence. It is possible to locally expand and/or compress this chronological sequence. This can allow the use of a common classifier function to select the compliance class for different sets of breathing information that should match different breathing and/or breath-holding patterns, e.g., having a different breath-holding time.

In a simple example, the compliance class can be determined by first determining a single value from the breathing information and then comparing this single value to a threshold. It is, e.g., possible to determine the variation of the measurements of the chronological sequence during at breath holding interval with the threshold. It was however recognized, that the robustness of the classification can be improved, when multiple breathing pattern parameters that are determined from the chronological sequence are taken into account.

When the breathing information describes a chronological sequence of multiple measurements concerning a respective breathing status, a mapping function can be used to map these measurements to a number of breathing pattern parameters, wherein the number of breathing pattern parameters is smaller than the number of measurements in the chronological sequence. In other words, the mapping function maps the chronological sequence to an, in particular approximate, representation of a lower dimensionality. The number of breathing pattern parameter can, e.g., be less than 50 or less than 20. The number of measurements in the chronological sequence can, e.g., be a larger than 100 or larger than 1000. In particular the number of breathing pattern parameters can be larger than 3 or larger than 10. It was found, that the given intervals for this number allow for a robust classification, while keeping the complexity of the algorithms relatively low. The discussed mapping can comprise the local expansion and/or compression of the time sequence that was already discussed above and/or a resampling of the chronological sequence as a preparatory step, before the actual mapping to the breathing pattern parameters is performed.

A classifier used to select the compliance class can therefore operate on a lower number of input parameters, namely the breathing pattern parameters, therefore reducing the complexity of the classifier. This can especially be advantageous, when the classifier is formed by a model that is trained by machine learning, as discussed in more detail later. In this case, lowering of the dimensionality of the input data can lower the number of free parameters of the model, which need to be determined during the training of the model, e.g., by reducing the number of nodes in the input layer of a neural network and therefore typically also in the following layers. This will, in turn, reduce the amount of training data required to train the model and also lower the resources required for the training of the model and for the use of the trained model.

The mapping function can be or comprise a first trained machine learning model that is based on multiple training data sets comprising a respective reference sequence of measurements concerning a respective breathing status of a respective patient.

The mapping function is preferably designed in such a way, that a good approximation of the chronological sequence can be reconstructed from the breathing pattern parameters, even when a low number of breathing pattern parameters, e.g., less than 50 or less than 20, e.g., 16 breathing pattern parameters are used, while the chronological sequence can, e.g., comprise hundreds or thousands of measurements.

To allow for such a strong reduction in dimensionality while keeping the loss of information low, it is advantageous to use prior knowledge about which kinds of chronological sequences can be expected. A relatively easy and robust way to take such prior knowledge into account is the use of a model that is based on training data concerning previously measured chronological sequences that can be used as reference sequences.

The training of the first trained machine learning model can be performed outside of the claimed method. In other words, the computer implemented method according to the present invention can use a first trained machine learning model based on the discussed training. The method can therefore, in particular, not comprise the training itself.

In general, a trained machine learning model mimics cognitive functions that humans associate with other human minds. In particular, by a training based on training data the machine learning model is able to adapt to new circumstances and to detect and extrapolate patterns. Another term for "trained machine learning model" is "trained function".

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of machine learning models can be adapted iteratively by several steps of training. In particular, within the training a certain cost function can be minimized. In particular, within the training of a neural network the backpropagation algorithm can be used.

In particular, a machine learning model can comprise a neural network, a support vector machine, a decision tree and/or a Bayesian network, and/or the machine learning model can be based on k-means clustering, Q-learning, genetic algorithms and/or association rules. In particular, a neural network can be a deep neural network, a convolutional neural network, or a convolutional deep neural network. Furthermore, a neural network can be an adversarial network, a deep adversarial network and/or a generative adversarial network.

The first trained machine learning model can in particular be based on manifold learning. Manifold learning is an approach to non-linear dimensionality reduction and therefore particularly suited for the task of reducing the dimensionality of the chronological sequence. Algorithms for manifold learning are based on the idea, that the dimensionality of many data sets is only artificially high, e.g., in the example of the breathing information due to noise and very minor variations in the breathing patterns that are not really relevant for the medical imaging.

The first trained machine learning model can be based on a training of an autoencoder. Shallow learning and therefore, e.g., at neural network with a low number of layers can typically be used, since a relatively low amount of input data is processed and the amount of output data is also small, especially compared to a typical image processing task. Therefore, it is, e.g., possible to train the model using a relatively simple backpropagation of error approach, e.g. by using a gradient descent.

An autoencoder is a type of artificial neural network used to learn an efficient encoding of unlabeled data. An autoencoder comprises two partial models, namely an encoder that transforms the input data to a sparse representation, namely the breathing pattern parameters, and a decoder that aims to recreate the input data, namely the chronological sequence, from the encoded representation. The autoencoder can be trained to minimize an error between the output sequence regenerated by the decoder and the original input, namely the chronological sequence.

Once the training of the autoencoder is finished, the partial model of the autoencoder forming the encoder can then be used as the first trained machine learning model in the mapping function.

In an advantageous embodiment, the first trained machine learning model can additionally be based on a respective reference class that is assigned to the respective reference sequence, and a cost function depending on a measure of the distance between

- on the one hand, the breathing pattern parameters for different reference sequences having the same assigned reference class, and/or
- on the other hand, the breathing pattern parameters for different reference sequences having a mutually different assigned reference class.

As discussed above, the cost function can additionally depend on the error between the respective reference sequence and the output sequence reconstructed by the trained autoencoder that comprises the first trained machine learning model as the encoder. Therefore, the method can, e.g., be implemented by extending the cost function used while training the auto encoder by additional terms that take the output of the encoder into account. Therefore, a minimization of the cost function can minimize the measure of the distance between points in the breathing pattern parameter space to which reference sequences with the same assigned reference class are mapped and/or maximize the measure of the distance between points in the breathing pattern parameter space to which reference sequences with mutually different assigned reference classes are mapped. To take both of these measures into account, the additional terms can, e.g., describe a triplet loss.

When the respective reference class can be considered to match or at least have a defined relationship with the compliance class that would be assigned to the associated reference sequence, the inclusion of the discussed measures of distance in the cost function can lead to a first trained machine learning model and therefore a mapping function that maps chronological sequences, for which the same compliance class should be selected, into close proximity to each other in the breathing pattern parameter space and/or that maps chronological sequences, for which different compliance classes should be selected into separate areas of the breathing pattern parameter space.

In the simplest case, the breathing pattern parameter space, that corresponds to a geometrical space spanned by the breathing pattern parameters, can then be segmented into distinct areas, wherein each area is assigned a different compliance class. It is, e.g., possible to use a mapping function that maps all or essentially all chronological sequences that match a desired breathing and/or breath-holding pattern to an area delimited by a sphere or a polyhedron. The compliance class can then be determined by checking, whether the breathing pattern parameters for the respective chronological sequence lie within that area.

It was, however, found, that the accuracy of the determination can often be even further improved, when a classifier, that is trained by machine learning, is used to determine the compliance class from the breathing pattern parameters. This will be discussed in more detail later. The robustness of such a trained classifier is however typically improved by using the mapping function discussed above to determine the breathing pattern parameters.

In the simplest case, the reference class can be contained in the respective training dataset and can, e.g., be based on a manual classification by a medical expert that can either be purely based on the respective reference sequence itself or that can, additionally or alternatively, take the image quality of a medical image acquisition into account, during and/or before which the reference sequence was acquired.

The reference class assigned to the respective reference sequence can be based on a measure for an image quality of a medical image dataset acquired during a reference imaging procedure, for which the respective reference sequence was acquired, and/or on a clustering, in particular on a k-means clustering, of the reference sequences. Both approaches allow for an automatic determination of the respective reference class, in particular without requiring input from a medical expert.

By performing a clustering on the reference sequences, similar reference sequences can be grouped into two or more clusters. In the simplest case, one of these clusters can indicate a sufficient compliance of the patient during the acquisition of the respective reference sequence and the other cluster can indicate an insufficient compliance.

It is however also possible to use more than two clusters, e.g., to allow for different types of non-compliance and/or to distinguish between a degree, to which the patient follows instructions, and therefore, e.g., the accuracy of the timing of a breath hold, and the quality of the breath hold itself. Multiple clusters can also allow for a separation between multiple quality classes and allow, e.g., to distinguish between a good, a sufficient and an insufficient quality of the breath hold, of the accuracy with which the patient follows instructions and/or of the overall quality of the reference sequence.

Each of the reference sequences can be acquired during a previous medical imaging procedure, in particular during the same type or at least a comparable type of medical imaging procedure to the medical imaging procedure that is to be monitored and/or controlled. In this case, the reference class can additionally or alternatively be based on a determined measure for the image quality of the medical image dataset acquired during the respective imaging procedure. It can therefore be determined, if the actual breathing and/or breath-holding pattern represented by the reference sequence is well suited to provide a sufficient image quality.

The proposed clustering and/or image quality analysis can reduce the effort necessary to provide the first trained machine learning model, since a manual classification of the potentially large number of reference sequences can be avoided.

A cluster information concerning multiple clusters in the parameter space spanned by the breathing pattern parameters can be received or determined, wherein the cluster information is based on a cluster analysis, in particular based on a k-means clustering, of multiple sets of breathing pattern parameters, wherein the respective set of the breathing pattern parameters is determined by a mapping of a respective sample sequence of measurements concerning a respective breathing status of a respective patient to this parameter space using the mapping function, wherein each of the clusters corresponds to one of the possible compliance classes, wherein the compliance class for the chronological sequence is determined, based on the cluster information, by determining to which one of the multiple clusters the set of breathing pattern parameters, to which the chronological sequence is mapped by the mapping function, belongs.

As already discussed, the mapping function can be designed or parametrized in such a way, that chronological sequences that, e.g., correspond to the same degree of compliance, e.g., to the same breath hold quality and/or the same degree to which the patient follows given instructions, are mapped closer to each other into the parameter space of the breathing pattern parameters than chronological sequences corresponding to different degrees of compliance. The described cluster analysis therefore essentially provides a classification via geometrical segmentation of the parameter space, that was already discussed. Compared to other approaches for such a geometrical segmentation, cluster analysis can allow for a more robust classification.

In principle it is possible to perform the full cluster analysis for each new chronological sequence, for which a compliance class is to be selected. To achieve this, the clusters can be provided by performing the cluster analysis on a group of sequences that comprises the chronological sequence to be analyzed and the sample sequences.

When a sufficient number of sample sequences is used, the influence of the addition of the chronological sequence to the sample sequences will only influence the position and size of the various clusters to a negligible degree. It is therefore possible, to provide a cluster information that describes fixed clusters as part of a classification algorithm that is used to select the classification based on the breathing pattern parameters determined for the respective chronological sequence. It is, e.g., possible to use the coordinates of the centers of the determined clusters as the cluster information and only use the distance of the coordinates determined by the application of the mapping function to the chronological sequence to the centers of the different clusters to assign the chronological sequence to the closest cluster and therefore to the compliance class assigned to this cluster.

The determination of the clusters and therefore the cluster information and therefore essentially the training of the classification algorithm can be performed outside of the claimed method. In other words, the computer implemented method according to the present invention can use a classification algorithm based on the discussed cluster analysis. It can therefore, in particular, not comprise this cluster analysis.

The sample sequences of measurements concerning a respective breathing status of a respective patient can be identical or at least partially comprise the reference sequences. It can however be advantageous, to provide the sample sequences or at least some of the sample sequences as additional training data beyond the training data used to train at the first trained machine learning model, to ensure a complete or at least a partial independence between the training of the first trained machine learning model and therefore the parametrization of the mapping function and the cluster analysis.

It should be noted, that the discussed cluster analysis can also be used, when the mapping function is not parametrized by machine learning and/or trained without the use of training data.

The clustering of the reference sequences, that was discussed earlier, can depend on a dynamic time warping distance between the different reference sequences or between partial sequences of the different reference sequences as a distance measure. Additionally or alternatively, the cluster analysis of the multiple sets of breathing pattern parameters can depend on a dynamic time warping distance between different reconstructed sequences, which are reconstructed from the respective set of breathing pattern parameters using a decoder, or between partial sequences of the different reconstructed sequences as a distance measure.

Dynamic time warping (DTW) is a well-known algorithm for measuring the similarity between two temporal sequences, which may vary in speed. Such a distance measure can be determined for two temporal sequences by distorting one of the temporal sequences in time, using a warping path, to minimize the sum of the local distances between the sequences. The sum of the remaining local distances, that results for an optimum warping path, can then be used as the time warping distance.

The use of such distance measure can be advantageous, since, even in the case of an optimum compliance of the patient to, e.g., breathing instructions, parts of the resulting breathing pattern can vary in speed. It is, e.g., possible, that different patients require different amounts of time to breathe in and/or out. Such differences can be compensated by using a distance measure based on dynamic time warping for the full respective sequence or for parts of the sequence, for which such a variance is expected and/or will likely not or hardly influence the medical imaging procedure.

The compliance class can be selected by using a classifier that processes the breathing pattern parameters as input data, wherein the classifier is or comprises a second trained machine learning model. As already discussed above, the use of the second trained machine learning model to select the compliance class can improve the accuracy of the classification, e.g., over a classification based on a geometrical segmentation of the breathing pattern parameter space that was discussed above. Due to the improvement of this classification, the accuracy of the monitoring and/or of the control of the medical imaging procedure is also improved.

The identification of this trained machine learning model as the second trained machine learning model is only chosen to distinguish the second trained machine learning model and features discussed with respect to this model from the first machine learning model and its features. The designation as the second trained machine learning model does not require the previously discussed first trained machine learning model to be used. It is therefore possible, that the inventive method uses only the first trained machine learning model, only the second trained machine learning model or both of these trained machine learning models.

The discussion of general features and possible implementations of a trained machine learning model that was given in the context of discussing the first trained machine learning model also applies for the second trained machine learning model.

The second trained machine learning model can, e.g., be based on a relatively simple neural network, since a relatively small amount of input data, e.g., less than 20 breathing pattern parameters, are processed. It is, e.g., possible to only use a single hidden layer or a few, e.g., less than 3 or less than 5, hidden layers. Preferably at least some of the layers are fully connected layers.

The second trained machine learning model can be based on a supervised training using multiple training datasets, wherein each training dataset can comprise a set of breathing pattern parameters and the reference compliance class that should be selected as the compliance class for this set of breathing pattern parameters. The training can be performed using a backpropagation of error, e.g., by a gradient descent approach. The breathing pattern parameters can be directly given or they can be determined on the fly from a respective reference sequence using the mapping discussed above.

The reference compliance class can be manually determined by a medical expert by reviewing the breathing pattern parameters and/or a reference sequence from which the respective breathing pattern parameters are determined and/or image data that was acquired in the context of such a reference sequence and/or a reconstructed reference sequence that can, e.g., be generated by processing the breathing pattern parameters using the decoder of the trained Autoencoder discussed above.

In a preferred embodiment, the selection of the reference compliance class for the respective training dataset is at least partially automated. It is, e.g., possible to perform a clustering, in particular a k-means clustering, of the reference sequences used to provide the reference breathing pattern parameters to allow the medical expert to review and classify such clusters. This can reduce the work load required for generating the training data.

Training data that is used to train the first and/or second trained machine learning model and/or a classifier based on cluster analysis can in particular be selected and/or curated and/or labeled by medical experts.

The medical imaging procedure can be controlled by starting and/or stopping and/or continuing and/or restarting an imaging sequence and/or issuing a breathing command to the patient, when a respective trigger condition, that depends on the selected compliance class, is fulfilled, and/or by adjusting at least one parameter of the imaging sequence based on the selected compliance class.

The compliance class can be determined at different times during the imaging sequence and/or before the start of the imaging sequence. It is, in particular, possible to evaluate the compliance class multiple times during the medical imaging procedure.

In an example, the patient can be instructed to breathe out, then completely breath in and then hold his or her breath, until the measurement sequence is finished. These instructions also define the desired breathing and breath-holding pattern. In this example, the actual imaging sequence can, e.g., only be started when the compliance class indicates that the patient complied with the first part of the desired breathing and breath-holding pattern, up until the initial breath-holding, sufficiently well.

When the compliance a class indicates an insufficient compliance, the imaging sequence can, e.g., be paused or stopped and the instructions to the patient can be repeated. If the compliance class, e.g., indicates, that the compliance of the patient seems to be a very poor, optionally the parametrization the imaging sequence can be changed to, e.g., switch to an acquisition that requires less compliance of the patient and/or that require no breath-hold or a shorter breath-hold. During the imaging sequence, e.g., during the acquisition of magnetic resonance data, the compliance class can be repeatedly determined using a respective updated breathing information. When the compliance class indicates an insufficient compliance of the patient, e.g., an insufficient breath-hold, the imaging sequence can be directly aborted. The breathing instructions can then be repeated and the measurement sequence can be restarted, once the patient has followed these breathing instructions sufficiently well according to the newly determined compliance class.

Optionally at least one parameter of the imaging sequence can be modified before the imaging sequence is started or restarted, e.g., to require a shorter breath-hold time than the original imaging sequence. The decision, if such a modification should be performed, can, e.g., depend on the compliance class that was determined, while the previous iteration of the imaging sequence was running. This compliance class can, e.g., allow for a distinction between an inability of the patient to perform a sufficiently long breath-hold and a breathing pattern that seems to indicate, that the patient simply did not comply with the instructions.

The imaging sequence can, in particular, comprising the sequence of steps that are performed during the medical imaging procedure. During a respective step one or more components of the medical imaging device used in the medical imaging procedure can be controlled. In a first example, the medical imaging procedure can, e.g., be or comprise magnetic resonance imaging and the imaging sequence can, e.g., comprise the generation of one or more excitation pulses, changes to gradient fields and/or the reception of signals, e.g., of a spin echo, from the patient. In a second example, in the case of a computed tomography, the imaging sequence can comprise the acquisition of several x-ray images for different projection directions.

The selection of the compliance class can additionally depend on at least one additional parameter, namely a timing of instructions concerning the desired breathing and/or breath-holding pattern given to the patient and/or on an imaging sequence used to control at least one medical imaging device during the medical imaging procedure and/or on a size and/or a weight of the patient and/or on a diagnostic information concerning the patient. The diagnostic information can, e.g. be a diagnostic code, in particular an ICD- or SCD code.

The given additional parameters can directly influence the desired breathing and/or breath-holding pattern or indirectly influence the desired pattern by modifying the imaging sequence. To take this influence into account, it is possible to pre-process the breathing information, e.g., to perform a local time scaling of the acquired chronological sequence.

Additionally or alternatively, various functions used during the determination of the compliance class can be adjusted based on the at least one additional parameter. It is, e.g., possible to use a different mapping function and/or a different classifier and/or a different clustering for the cluster analysis depending on the value of the at least one additional parameter.

Additionally or alternatively, the at least one additional parameter can form an additional input parameter for at least one of the functions used for selecting the compliance class. It is, in particular, possible to take these additional input parameters into account, when training the first and/or the second trained machine learning model.

The invention also concerns a method for providing the first trained machine learning model for the computer-implemented method for monitoring and/or controlling a medical imaging procedure discussed above, the method comprising:

- receiving multiple training data sets comprising a respective reference sequence of measurements concerning a respective breathing status of a respective patient as input training data,
- training a machine learning model based on the input training data to determine the first trained machine learning model, and
- providing the first trained machine learning model.

Various details and advantageous embodiments of the training of the first machine learning model were already discussed above and these details and the features of the various embodiments discussed above can also be used in the computer-implemented method for providing the first trained machine learning model with the advantages given above and vice versa.

It should especially be noted, that the first machine learning model can be trained as the encoder of an Autoencoder and that it is therefore possible to use unsupervised training to train the Autoencoder and therefore the first trained machine learning model.

As discussed above, it can however be advantageous to provide a respective reference class as additional training data and to train the machine learning model in additional dependence on this additional training data. The respective reference class can be determined on the fly during the training or be provided as part of the respective training dataset. Both of these approaches were already discussed above.

Additionally, the invention concerns a computer-implemented method for providing the second trained machine learning model for the computer-implemented method for monitoring and/or controlling a medical imaging procedure discussed above, the method comprising:

- receiving multiple sets of breathing pattern parameters as input training data,
- receiving a respective associated reference compliance class for each one of the sets of breathing pattern parameters as output training data,
- training a machine learning model based on the input training data and the output training data to determine the second trained machine learning model, and
- providing the second trained machine learning model.

Various details and advantageous embodiments of the training of the second machine learning model were already discussed above and these details and the features of the various embodiments discussed above can also be used in the computer implemented method for providing the second trained machine learning model with the advantages given above and vice versa.

As discussed above, the second trained machine learning model can be trained using a supervised training using multiple training datasets, each comprising a set of breathing pattern parameters and the reference compliance class that should be determined for this set of breathing pattern parameters. The training can, e.g., be performed using a backpropagation of error, in particular, by a gradient descent approach.

Additionally, the invention concerns a data processing system, that is designed to carry out at least one of the previously discussed computer-implemented methods. The data processing system can, e.g., be a desktop computer, a server or be implemented as a cloud solution. Additionally or alternatively, it can be integrated into a medical imaging device. The data processing system can have hardware and/or software interfaces for receiving the various inputs discussed above.

The invention also concerns a computer program that comprises instructions to carry out at least one of the previously discussed computer-implemented methods when the program is executed on a data processing system.

Additionally, the invention concerns a computer-readable medium comprising this computer program.

Other objects and features of the present invention will become apparent from the following detailed description considered in conjunction with the accompanying drawings. The drawings, however, are only principle sketches designed solely for the purpose of illustration and do not limit the invention. The drawings show:

Fig. 1   a flowchart of an exemplary embodiment of the computer-implemented method for monitoring and/or controlling a medical imaging procedure on a patient according to the present invention,

Fig. 2   exemplary data structures that can be used in the method according to fig. 1,

Fig. 3   an exemplary embodiment of the data processing system according to the present invention,

Fig. 4   an example of a machine learning model,

Fig. 5   a flowchart of an exemplary embodiment of the computer-implemented method for providing the second trained machine learning model according to the present invention,

Fig. 6    a flowchart of an exemplary embodiment of the computer-implemented method for providing the first trained machine learning model according to the present invention, and

Fig. 7    a flow chart of a further embodiment of the selection of the compliance class in the computer-implemented method for monitoring and/or controlling a medical imaging procedure on a patient according to the present invention.

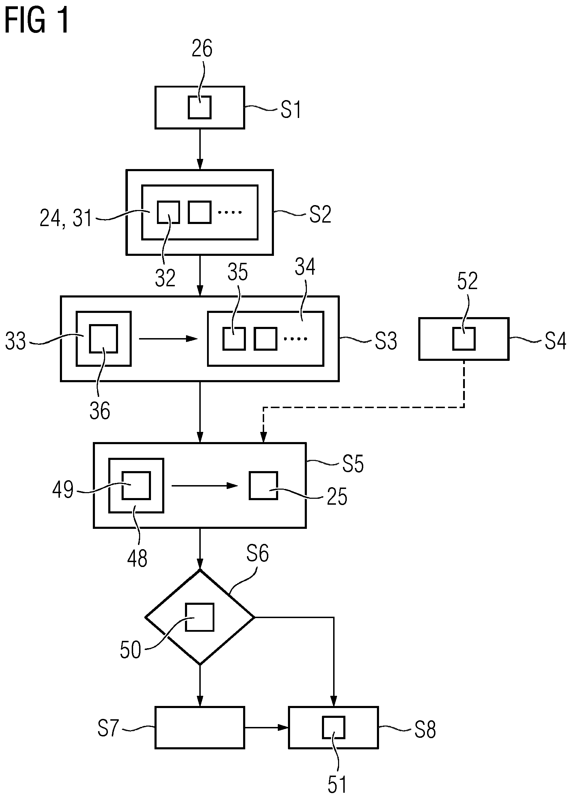

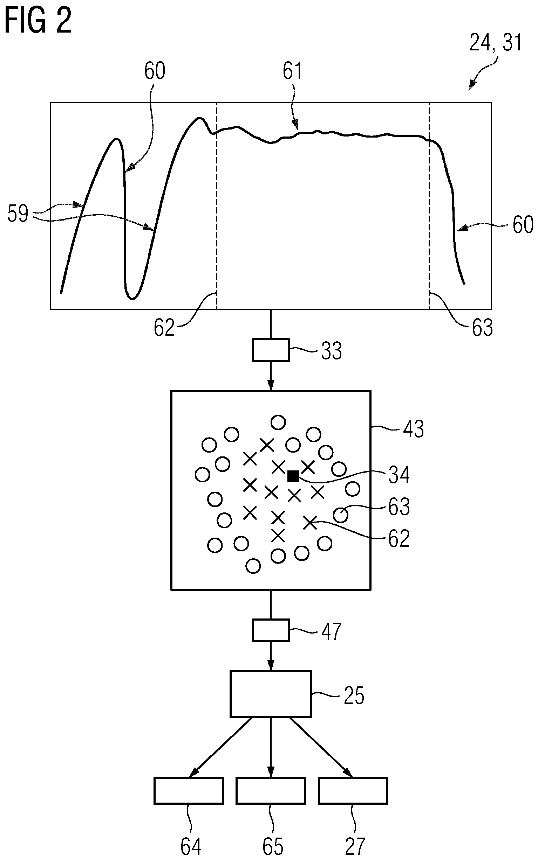

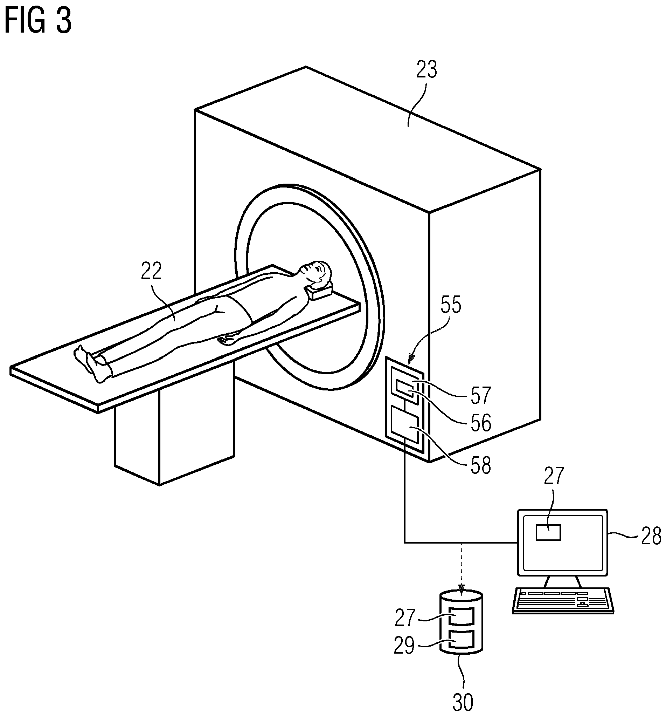

Fig. 1 shows a flowchart of a computer-implemented method for monitoring and/or controlling a medical imaging procedure on a patient. This method will be described in conjunction with fig. 2, which shows some of the data structures that can be used in this method, and with fig. 3 that shows a typically usage situation for the method and the data processing system 55 used to implement this method.

In the example shown in fig. 3, the medical imaging is performed by a medical imaging device at 23, e.g., a magnetic resonance tomograph, on a patient 22. The method is implemented by the data processing system 55 that comprises a programmable processor 58 and the memory 57 storing a program 56 that implements the method. In the example, the data processing system 55 is networked to a remote storage 30 to store the acquired medical image data 29 and to a desktop computer 28 that allows a user to control and monitor the medical imaging procedure.

The medical imaging procedure requires the patient 22 to closely follow a desired breathing and/or breath-holding pattern 26, that can, e.g., be communicated to the patient 22 using internal loudspeakers (not shown) of the medical imaging device 23 in a step S1. The provision of these instructions can be fully automatic and a part of the imaging sequence used to acquire in medical image data 29.

As already discussed in the general part of the description, an insufficient compliance of the patient 22 with these instructions can lead to medical image data 29 that is potentially unusable and can therefore require a new image acquisition at a later point in time. To allow for an earlier detection of such non-compliance, the discussed method automatically monitors the compliance of the patient 22.

In step S2 a breathing information 24 concerning a breathing pattern of the patient 22 is received by the data processing system 55. In the example, the breathing information 24 is acquired using the medical imaging device 23 itself, namely using a pilot tone to determine the movement of the diaphragm of the patient 22. Alternatively, the breathing information could be acquired using a dedicated sensor. Various suitable sensors where already discussed in the general part of the description.

The breathing information 24 describes a chronological sequence 31 of multiple measurements 32 concerning a respective breathing status, e.g. the position of the diaphragm or the extension of a chest belt over time. A graphical representation of an exemplary chronological sequence 31 is shown in fig. 2. The time and therefore in the number of the number of the sample is plotted on the x-axis and the y-axis corresponds to the breathing motion of the patient 22.

The rising flanks 59 correspond to an inhaling by the patient 22 and the falling flanks 60 corresponds to an exhaling. The section 61 is essentially flat and corresponds to a breath hold. The actual image acquisition is performed between the times 62 and 63 and therefore during the breath hold in the example. The example shown in fig. 2 is therefore an example for a good compliance of the patient 22 with the desired breathing and/or breath-holding pattern 26. A bad compliance might, e.g., be detected when large changes of the curve are present between the times 62 and 63 and/or by when there is no full breathing cycle before the time 62.

Since the example shown in fig. 1 comprises several advantageous but optional features, the following gives a brief overview over the remaining steps of the method, before the individual steps will be discussed in detail:
In steps S3 to S5 a compliance class 25 is selected from multiple possible compliance classes based on the breathing information 24. At least one of the possible compliance classes corresponds to a compliance of the acquired breathing information 24 with the desired breathing and/or breath-holding pattern 26.

In the steps S6 to S8 the medical imaging procedure is controlled depending on the selected compliance class 25. Additionally, a non-compliance information 27 can be given to the user, e.g., via the desktop computer 28, and/or stored with the medical image data 29, when the selected compliance class 25 does not indicate a compliance of the acquired breathing information 24 with the given desired breathing and/or breath-holding pattern 26.

Continuing with the detailed discussion of the individual steps of the example, in step S3 a mapping function 33 is used to map the measurements 32 to a set 34 of breathing pattern parameters 35. The mapping function 33 is chosen to output to a lower number of breathing pattern parameters 35 than the number of measurements 32 in the chronological sequence 31. Therefore, the mapping function 33 reduces the dimensionality of the breathing information 24.

The mapping function 33 can, e.g., map the measurements 32 into a 16-dimensional breathing pattern parameters space 43. An exemplary breathing pattern parameter space 43 is shown in fig. 2, wherein a two-dimensional breathing pattern parameter space 43 is used to simplify the illustration. The position defined by the breathing pattern parameters 34 is marked in fig. 2.

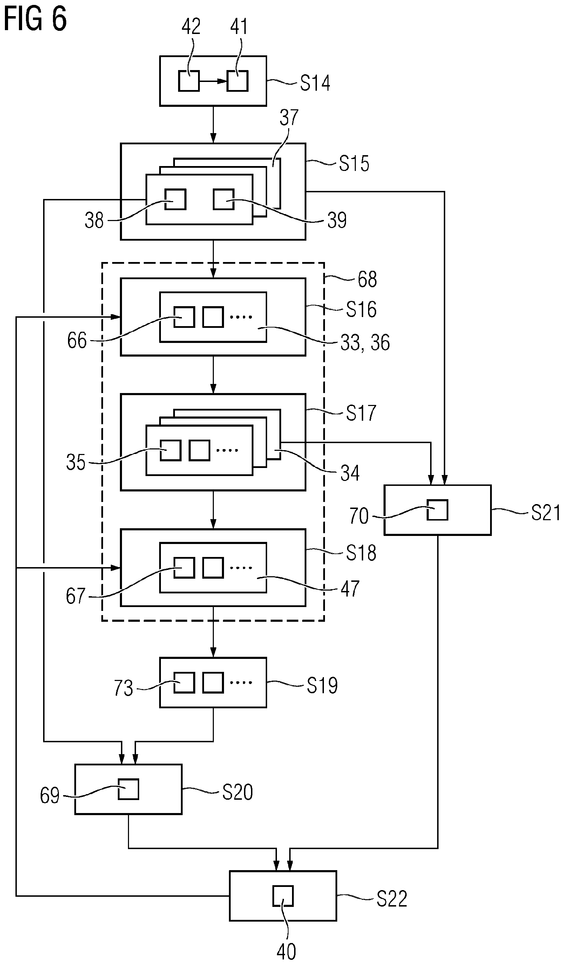

In the example the mapping function 33 is implemented by a first trained machine learning model 36. Further details concerning the first trained machine learning model 36 will be given later with reference to fig. 6 that concerns the training of this model.

It should however already be noted, that the mapping function 33 is designed and parametrized in such a way in the example, that sets 62 of breathing pattern parameters 35, that correspond to chronological sequences having a first compliance class that indicates a good compliance and that are marked by a cross in fig. 2 are grouped closely together, while sets 63 of breathing pattern parameters 35 corresponding to a second compliance class that indicates a bad compliance and that are marked by a circle in fig. 2 are separated from the sets 60 due to the mapping.

In step S4 additional parameters 52 can optionally be provided, that can, e.g., influence, which one of multiple possible classifiers 48 is used in step S5. The additional parameters can, e.g., concern diagnostic information concerning the patient 22. Other potentially relevant additional parameters where already discussed in the general part of the description.

Such additional parameters can, e.g., influence, what degree of compliance is tolerated and/or influence the imaging sequence and therefore, e.g., the required breath-holding time. Alternatively or additionally, it would also be possible, to perform the mapping in step S3 in dependence on these additional parameters 52.

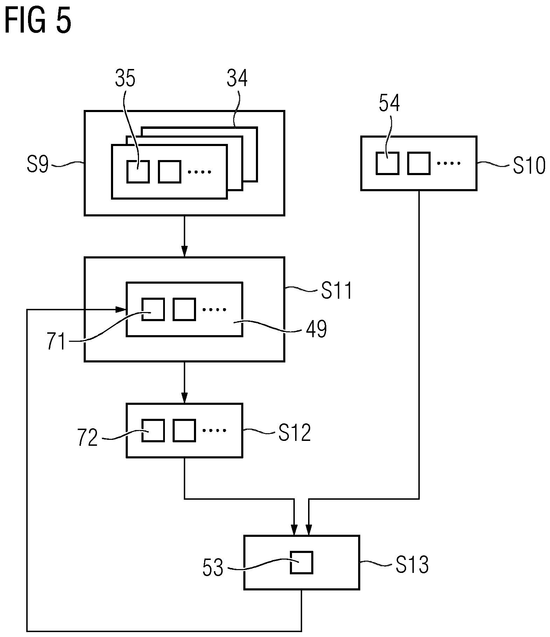

In step S5 the compliance classifier 25 is selected from multiple options based on the previously determined breathing pattern parameters 35. In the example, the compliance class 25 is selected by using a classifier 48 that processes the breathing pattern parameters 35 as input data, wherein the classifier 48 is implemented by a second trained machine learning model 49. Further details concerning the second trained machine learning model 49 will be given later with reference to fig. 5 that concerns the training of this model.

In step S6 a trigger condition 50 that depends on the selected compliance class 25 is evaluated. In the example, the trigger condition 50 is fulfilled, when the selected compliance class 25 does not indicate a compliance of the acquired breathing information 24 with the given desired breathing and/or breath-holding pattern 26.

When the trigger condition 50 is fulfilled in step S6, multiple actions can be triggered in step S7 to handle the non-compliance of the patient 22 with the breathing and/or breath-holding pattern 26. As already discussed in the general part of the description, different actions can be used depending on whether the compliance class is selected before the imaging sequence has started, during the imaging sequence or after the imaging sequence is finished.

In all cases, a non-compliance information 27 can be output to a user, e.g., via the desktop computer 28. When the image acquisition is already finished, the non-compliance information 27 can also be stored with the acquired medical image data 29, to inform users of this data, that the medical image data 29 is potentially compromised.

Additionally or alternatively, an imaging sequence that is already running can be stopped and therefore aborted in the step S7 to allow for a faster repetition of the sequence, especially after issuing a breathing command to the patient 22.

It should be noted, that in other embodiments there could also be actions that only trigger, when the selected compliance class indicates a good compliance of the patient 22. It is, e.g., possible, to only start or resume an imaging sequence, when such a compliance class is selected.

In step S8, the medical imaging procedure is adjusted depending on the selected compliance class 25. It is, e.g., possible to the adjust at least one parameter 51 of the imaging sequence based on the selected compliance class 25. This can be especially useful, when multiple levels of compliance are detected by selecting from more than two possible compliance classes. In this case, multiple compliance classes can allow for the imaging sequence or to continue. It can however be useful, to adjust the imaging sequence when the selected compliance class 25 does indicate a sufficient, but not optimal compliance. In this case it can, e.g., be possible to modify the imaging sequence to require a shorter breath-hold time, to make it easier for the patient 22 to comply with the desired breathing and/or breath-holding pattern 26.

As shown schematically in fig. 2, the possible actions triggered by the selection of the compliance class 25 can, e.g., be separated into the generation of the control signal 64 to control the imaging sequence, the giving of breathing instructions 65 to the patient 22 and the outputting of the non-compliance information 27.

In the embodiment according to fig. 1, both the mapping function 33 used in the step S3 and the classifier 48 used in step S5 are implemented as a trained machine learning models 36, 49. The respective trained machine learning model 36, 49 can in particular have the structure of a neural network.

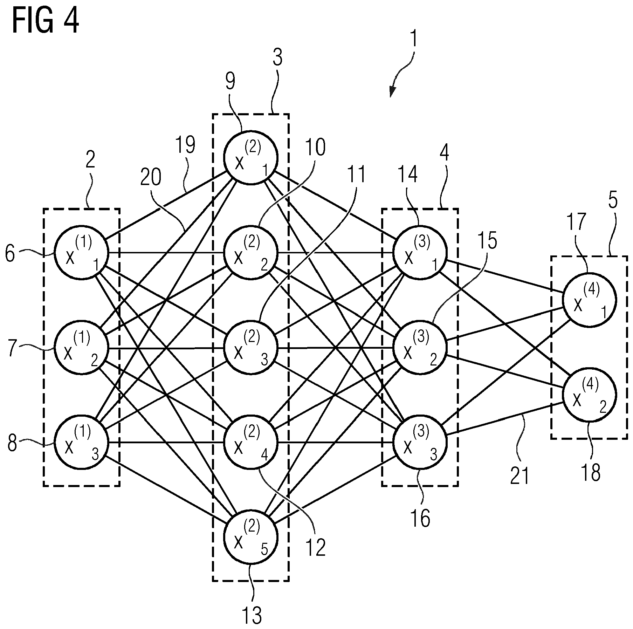

To illustrate the features and working of such a neural network, fig. 4 displays an embodiment of an artificial neural network 1. For simplicity's sake, a relatively simple network is shown and discussed.

For an implementation of the first trained machine learning model 36, a larger network can be used, that has an input note 6 - 8 for each measurement of the chronological sequence 31 and an output node 17, 18 for each of the breathing pattern parameters 35.

For an implementation of the second trained machine learning model 49, the number of input nodes 6 - 8 can correspond to the number of a breathing pattern parameters 35 and the number of output nodes can, e.g., correspond to the number of possible compliance classes. Output nodes can then, e.g., output the probability, that an input corresponds to the respective compliance class and the class with the highest probability can be selected as the selected compliance class 35.

Alternative terms for "artificial neural network" are "neural network", "artificial neural net" or "neural net".

The artificial neural network 1 comprises nodes 6 - 18 and edges 19 - 21, wherein each edge 19 - 21 is a directed connection from a first node 6 - 18 to a second node 6 - 18. In general, the first node 6 - 18 and the second node 6 - 18 are different nodes 6 - 18. It is also possible that the first node 6 - 18 and the second node 6 - 18 are identical. For example, in Fig. 1 the edge 19 is a directed connection from the node 6 to the node 9, and the edge 20 is a directed connection from the node 7 to the node 9. An edge 19 - 21 from a first node 6 - 18 to a second node 6 - 18 is also denoted as "ingoing edge" for the second node 6 - 18 and as "outgoing edge" for the first node 6 - 18.

In this embodiment, the nodes 6 - 18 of the artificial neural network 1 can be arranged in layers 2 - 5, wherein the layers 2 - 5 can comprise an intrinsic order introduced by the edges 19 - 21 between the nodes 6 - 18. In particular, edges 19 - 21 can exist only between neighboring layers of nodes 6 - 18. In the displayed embodiment, there is an input layer 2 comprising only nodes 6 - 8 without an incoming edge, an output layer 5 comprising only nodes 17, 18 without outgoing edges, and hidden layers 3, 4 in-between the input layer 2 and the output layer 5. In general, the number of hidden layers 3, 4 can be chosen arbitrarily. The number of nodes 6 - 8 within the input layer 2 usually relates to the number of input values of the neural network, and the number of nodes 17, 18 within the output layer 5 usually relates to the number of output values of the neural network.

In particular, a (real) number can be assigned as a value to every node 6 - 18 of the neural network 1. Here, $x^{(n)}_i$ denotes the value of the i-th node 6 - 18 of the n-th layer 2 - 5. The values of the nodes 6 - 8 of the input layer 2 are equivalent to the input values of the neural network 1, the values of the nodes 17, 18 of the output layer 5 are equivalent to the output values of the neural network 1. Furthermore, each edge 19 - 21 can comprise a weight being a real number, in particular, the weight is a real number within the interval [-1, 1] or within the interval [0, 1] . Here, $w^{(m,n)}_{i,j}$ denotes the weight of the edge between the i-th node 6 - 18 of the m-th layer 2 - 5 and the j-th node 6 - 18 of the n-th layer 2 - 5. Furthermore, the abbreviation $w^{(n)}_{i,j}$ is defined for the weight $w^{(n,n+1)}_{i,j}$.

In particular, to calculate the output values of the neural network 1, the input values are propagated through the neural network 1. In particular, the values of the nodes 6 - 18 of the (n+1)-th layer 2 - 5 can be calculated based on the values of the nodes 6 - 18 of the n-th layer 2 - 5 by

$$x_j^{(n+1)} \;=\; f\left(\sum_i x_i^{(n)} \cdot w_{i,j}^{(n)}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network 1, wherein values of the input layer 2 are given by the input of the neural network 1, wherein values of the first hidden layer 3 can be calculated based on the values of the input layer 2 of the neural network 1, wherein values of the second hidden layer 4 can be calculated based in the values of the first hidden layer 3, etc.

In order to set the values $w^{(m,n)}_{i,j}$ for the edges 19 - 21, the neural network 1 has to be trained using training data. In particular, training data comprises training input data and training output data (denoted as $t_i$). For a training step, the neural network 1 is applied to the training input data to generate calculated output data. In particular, the training data and the calculated output data comprise a number of values, said number being equal to the number of nodes 17, 18 of the output layer 5.

In particular, a comparison between the calculated output data and the training data is used to recursively adapt the weights within the neural network 1 (backpropagation algorithm). In particular, the weights are changed according to

$$w'^{(n)}_{i,j} \;=\; w^{(n)}_{i,j} \;-\; \gamma \cdot \delta^{(n)}_j \cdot x^{(n)}_i$$

wherein $\gamma$ is a learning rate, and the numbers $\delta^{(n)}_j$ can be recursively calculated as

$$\delta_j^{(n)} \;=\; \left(\sum_k \delta_k^{(n+1)} \cdot w_{j,k}^{(n+1)}\right) \cdot f'\left(\sum_i x_i^{(n)} \cdot w_{i,j}^{(n)}\right)$$

based on $\delta^{(n+1)}_j$, if the (n+1)-th layer is not the output layer 5, and

$$\delta_j^{(n)} = \left( x_k^{(n+1)} - t_j^{(n+1)} \right) \cdot f' \left( \sum_i x_i^{(n)} \cdot w_{i,j}^{(n)} \right)$$

if the (n+1)-th layer is the output layer 5, wherein f' is the first derivative of the activation function, and $y^{(n+1)}_j$ is the comparison training value for the j-th node of the output layer 5.

Concrete examples for the training of the first and second trained machine learning model 36, 49 will now be discussed with reference to figures 5 and 6.

As shown in figures 5, the second trained machine learning model 49 can be trained using a supervised training. In step S9 multiple sets 34 of breathing pattern parameters 35 are received as input training data. The respective set 34 can be determined by processing a reference sequence of measurements concerning a respective breathing status of a respective patient by the mapping function 33 that was already discussed with reference to figure 1 and 2. The respective reference sequence can in particular be a chronological sequence of such measurements that was acquired while monitoring and/or controlling a respective previous medical imaging procedure.

In step S10 a respective associated reference compliance class 54 is received for each one of the sets 34 of breathing pattern parameters 35 as output training data. The reference compliance class corresponds to a compliance class, that should be selected by the second trained machine learning network 49 when it is applied to the respective set 34 of breathing pattern parameters 35 provided as input training data.

The reference compliance class 54 can, e.g., be manually assigned to the respective set 34 by a medical expert that evaluates the associated reference sequence and/or the results of the previous medical imaging procedure during which this reference sequence was acquired. Alternatively, the reference compliance class 54 can be automatically determined, e.g., by automatically determining a quality measure for the results of this previous medical imaging procedure and/or by the clustering of the reference sequences, as already discussed in the general part of the description.

To determine the parameters 71 parametrizing the second trained machine learning model, e.g. the weights for the different nodes, based on the input training data and the output training data, preliminary values of the parameters 71 are chosen in step S11 and the temporarily parametrized model is used to determine a probability distribution 72 for the possible compliance classes 25 for each of the sets 34.

In step S13 the cost function 53 is calculated, e.g., by adding an error term for each one of the sets, wherein the respective error term depends on the diversions of the probability distribution 72 from the correct distribution that is a delta distribution described by the associated reference compliance class.

The value of the cost function 53 is then minimized by iteratively varying the parameters 71, e.g., by using a gradient descent and a backpropagation of error.

As shown in fig. 6, the training of the first trained machine learning model 49 can be based on a slightly modified training of an autoencoder 68.

In step S15 multiple training data sets 37 that comprise a respective reference sequence 38 of measurements concerning a respective breathing status of a respective patient and a reference class 39 assigned to the respective reference sequence 38 are received. The respective reference sequence 38 can be acquired during a respective reference imaging procedure. The reference class 39 can especially correspond to a compliance class that should be assigned to the respective reference sequence 38, when the first trained machine learning model 49 is used as the mapping function 33 in the method discussed with reference to figure 1. In principle the reference class 39 could be manually assigned by a medical expert, e.g., after reviewing the respective reference sequence 38. In the example, the reference class 39 is however automatically determined based on a measure for an image quality 41 of a medical image dataset 42 acquired during a reference imaging procedure. the measure for the image quality 41 is determined in step S14 and used to provide the respective training data set 37 in step S15.

The steps S16 to S20 essentially correspond to the typical training of an autoencoder 68.

In step S16 the mapping function 33 and therefore the model that is trained to form the first trained machine learning model 36 is used to encode each one of the reference sequences 38 into a respective set 34 of breathing pattern parameters 35 that are provided in step S17.

The breathing pattern parameters 35 of each set 34 are then decoded in step S18 by a decoder 47 to provide a respective output sequence 73 in step S19 that should closely replicate the associated reference sequence 38 once of the training of the autoencoder 68 is finished.

The sum of the differences between each reference sequence 38 and the respective output sequence 73 that is generated by the autoencoder 68 from the respective reference sequence 38 is then used as the partial cost function 69 in step S20.

In a typical training of an autoencoder 68, the parameters 66, 67 of the autoencoder 68 and more specifically of the first trained machine learning model 36 and the decoder 47 would then be iteratively varied to minimize this partial cost function 69.

The training shown in figure 6 does however additionally take a further partial cost function 70 into account. The further partial cost function 70 is determined in step S21. The overall cost function 40 that can, in particular, be a sum or a weighted sum of the partial cost functions, can then be minimized to determine the parameters of 66, 67. As previously discussed, this minimization and therefore of the training can, e.g., be performed by a backpropagation of error, e.g., by gradient descent.

The partial cost function 70 depends on the distances of the positions of the various sets 34 of breathing pattern parameters 35 and therefore on the distance between positions associated with a respective one of the reference sequences 38 in the breathing pattern parameter space 43. It is, e.g., possible to use an Euclidean distance or any other distance measure.

The partial cost function 70 is chosen in such a way, that the cost can be lowered when positions associated with reference sequences 38 having the same associated reference class 39 are a grouped closer together. Additionally, the cost can be reduced, when positions associated with reference sequences 38 having mutually different reference classes 39 are spaced further part.

When the reference classes 39 at least approximately correspond to compliance classes 25, that should result when the respective reference sequence 38 is processed as discussed with reference to fig. 1, the use of the additional partial cost function 70 therefore trains the mapping function 33 to group chronological sequences 24 for which the same compliance class 25 should be selected in close vicinity to each other while separating them from chronological sequences 24 that are classified differently from each other in the breathing pattern parameters space 43. This allows for a more robust classification.

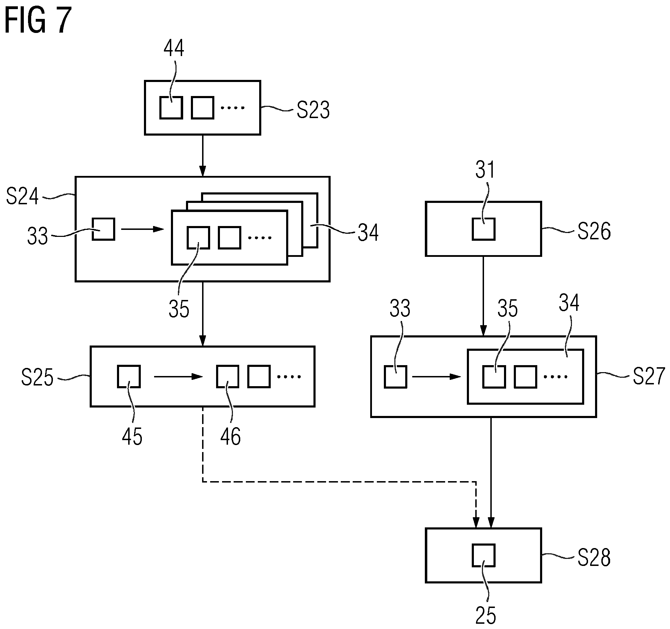

Figure 7 shows a flowchart of a further approach for determining the selected compliance class 25 for a given chronological sequence 31. The steps S26 to S28 that implement this selection and can, e.g., replace the steps S2 to S5 in the embodiment shown in fig. 1.

The steps S23 to S25 are training steps that are used to determine a cluster information that is used in a step S28 to select the compliance class 25. These steps are not necessarily included in the computer-implemented method for monitoring and/or controlling a medical imaging procedure and can be considered to form a separate method.

In step S23 multiple sample sequences 44 of measurements concerning a respective breathing status of a respective patient are provided. The provision of such sample sequences 44 is no different from, e.g., the provision of the reference sequences 38 that was already discussed.

In step S24 a respective set 34 of breathing pattern parameters 35 is determined by the mapping function 33. Step S24 is therefore essentially equivalent to the repeated performance of the step S3 shown in fig. 1 for each of the sample sequences 44.

In step S25 a cluster analysis 45, e.g., a k-means clustering, is performed to determine a cluster information concerning multiple clusters 46 in the parameter space 43 spanned by the breathing pattern parameters 35. Each of these clusters is assigned to one of the possible compliance classes. This can, e.g., be achieved by manually inspecting the various sample sequences 44 by a medical expert. It is however also possible to use an approach based on image quality analysis for images resulting from acquisition during which the respective sample sequence 44 was acquired. This approach was already discussed with respect to step S14 in fig. 6.

Parameters of the provided clusters 46, especially the center of the respective cluster, can then be provided as the cluster information used for the classification in step S28.

For this purpose, the chronological sequence 31 that is provided in step S26, is processed by the mapping function 33 in step S27 to provide a set 34 of breathing pattern parameters 35 and therefore a position in the breathing pattern parameter space 43.

Based on the relative position of this position to the positions of the various clusters 46 described by the cluster information, it can be determined, to which cluster the chronic logical sequence 31 belongs and therefore the compliance class 25 associated with this cluster 46 can be selected.

Although the present invention has been described in detail with reference to the preferred embodiment, the present invention is not limited by the disclosed examples from which the skilled person is able to derive other variations without departing from the scope of the invention.

Independent of the grammatical term usage, individuals with male, female or other gender identities are included within the term.

## Claims

**1.** Computer-implemented method for monitoring and/or controlling a medical imaging procedure on a patient (22), comprising the steps of:

- receiving a breathing information (24) concerning a breathing pattern of the patient (22),
- selecting a compliance class (25) from at least two possible compliance classes based on the breathing information (24), wherein at least one of the possible compliance classes corresponds to a compliance of the acquired breathing information (24) with a given desired breathing and/or breath-holding pattern (26), and
- on the one hand controlling the medical imaging procedure depending on the selected compliance class (25) and/or
- on the other hand outputting a non-compliance information (27) to a user and/or storing a non-compliance information (27) with an acquired medical image data (29), when the selected compliance class (25) does not indicate a compliance of the acquired breathing information (24) with the given desired breathing and/or breath-holding pattern (26).

2.  Computer-implemented method according to claim 1, **characterized in that** the breathing information (24) describes a chronological sequence (31) of multiple measurements (32) concerning a respective breathing status, wherein a mapping function (33) is used to map these measurements (32) to a number of breathing pattern parameters (35), wherein the number of breathing pattern parameters (35) is smaller than the number of measurements (32) in the chronological sequence (31).

3.  Computer-implemented method according to claim 2, **characterized in that** the mapping function (33) is or comprises a first trained machine learning model (36) that is based on multiple training data sets (37) comprising a respective reference sequence (38) of measurements concerning a respective breathing status of a respective patient.

4.  Computer-implemented method according to claim 3, **characterized in that** the first trained machine learning model is additionally based on

    - a respective reference class (39) that is assigned to the respective reference sequence (38), and
    - a cost function (40) depending on a measure of the distance between

       • on the one hand, the breathing pattern parameters (35) for different reference sequences (38) having the same assigned reference class (39), and/or
       • on the other hand, the breathing pattern parameters (35) for different reference sequences (38) having a mutually different assigned reference class (39).

5.  Computer-implemented method according to 4, **characterized in that** the reference class (39) assigned to the respective reference sequence (38) is based on a measure for an image quality (41) of a medical image dataset (42) acquired during a reference imaging procedure, for which the respective reference sequence (38) was acquired, and/or on a clustering, in particular on a k-means clustering, of the reference sequences (38).

6.  Computer-implemented method according to one of the claims 2 to 5, **characterized in that** a cluster information concerning multiple clusters (46) in the parameter space (43) spanned by the breathing pattern parameters (35) is received or determined, wherein the cluster information is based on a cluster analysis (45), in particular based on a k-means clustering, of multiple sets (34) of breathing pattern parameters (35), wherein the respective set (34) of the breathing pattern parameters (35) is determined by a mapping of a respective sample sequence (44) of measurements concerning a respective breathing status of a respective patient to this parameter space (43) using the mapping function (33), wherein each of the clusters (46) corresponds to one of the possible compliance classes (25), wherein the compliance class (25) for the chronological sequence (31) is determined by determining, based on the cluster information, to which one of the multiple clusters (46) the set (34) of breathing pattern parameters (35), to which the chronological sequence (31) is mapped by the mapping function (33), belongs.

7.  Computer-implemented method according to claim 5 or 6, **characterized**

    - **in that** the clustering of the reference sequences (38) depends on a dynamic time warping distance between the different reference sequences (38) or between partial sequences of the different reference sequences (38) as a distance measure, and/or
    - **in that** the cluster analysis (45) of the multiple sets (34) of breathing pattern parameters (35) depends on a dynamic time warping distance between different reconstructed sequences, which are reconstructed from the respective set (34) of breathing pattern parameters (35) using a decoder (47), or between partial sequences of the different reconstructed sequences as a distance measure.

8.  Computer-implemented method according to one of the claims 2 to 7, **characterized in that** the compliance class (25) is selected by using a classifier (48) that processes the breathing pattern parameters (35) as input data, wherein the classifier (48) is or comprises a second trained machine learning model (49).

9. Computer-implemented method according to one of the preceding claims, **characterized in that** the medical imaging procedure is controlled by

    - starting and/or stopping and/or continuing and/or restarting an imaging sequence and/or issuing a breathing command to the patient (22), when a respective trigger condition (50), that depends on the selected compliance class (25), is fulfilled, and/or
    - adjusting at least one parameter (51) of the imaging sequence based on the selected compliance class (25).

10. Computer-implemented method according to one of the preceding claims, **characterized in that** the selection of the compliance class (22) additionally depends on at least one additional parameter (52), namely a timing of instructions concerning the desired breathing and/or breath-holding pattern (26) given to the patient (22) and/or on an imaging sequence used to control at least one medical imaging device (23) during the medical imaging procedure and/or on a size and/or a weight of the patient (22) and/or on a diagnostic information concerning the patient (22).

11. Computer-implemented method for providing the first trained machine learning model (33) for the computer-implemented method according to claim 3 or for the computer-implemented method according to claim 3 and any one the claims 4 to 10, the method comprising:

    - receiving multiple training data sets (37) comprising a respective reference sequence (38) of measurements concerning a respective breathing status of a respective patient (22) as input training data,
    - training a machine learning model based on the input training data to determine the first trained machine learning model (33), and
    - providing the first trained machine learning model (33) .

12. Computer-implemented method for providing the second trained machine learning model for the computer-implemented method according to claim 8 or for the computer-implemented method according to claim 8 and either claim 9 or 10, the method comprising:

    - receiving multiple sets (34) of breathing pattern parameters (35) as input training data,
    - receiving a respective associated reference compliance class (54) for each one of the sets (34) of breathing pattern parameters (35) as output training data,
    - training a machine learning model based on the input training data and the output training data to determine the second trained machine learning model (49), and
    - providing the second trained machine learning model (49) .

13. Data processing system, **characterized in that** it is designed to carry out the computer implemented method according to one of the preceding claims.

14. Computer program, **characterized in that** it comprises instructions to carry out the computer-implemented method according to one of the claims 1 to 12 when the program is executed on a data processing system (55).

15. Computer-readable medium comprising the computer program (56) according to claim 14.

FIG 1

FIG 2

FIG 3

FIG 4

FIG 5

FIG 6

FIG 7

Europäisches
Patentamt

European
Patent Office

Office européen
des brevets

# EUROPEAN SEARCH REPORT

**Application Number**

EP 24 15 8895

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 109 998 487 A (SHANGHAI UNITED IMAGING HEALTHCARE CO LTD) 12 July 2019 (2019-07-12) * [0002], [0010], [0011], [0035], [0037], [0038], [0043], [0050], [0051], [0064], [0082] * ----- | 1-15 | INV. G16H30/20 G16H30/40 G16H40/63 |
| A | US 2023/248268 A1 (SA RUHAN [US] ET AL) 10 August 2023 (2023-08-10) * [0005], [0009], [0029], [0046], [0047], [0052] * ----- | 1-15 | |
| A | WO 2022/194666 A1 (KONINKLIJKE PHILIPS NV [NL]) 22 September 2022 (2022-09-22) * p.2, 3 * ----- | 1-15 | |
| A | US 2023/342914 A1 (BALAKRISHNAN KARTHIKAYAN [US] ET AL) 26 October 2023 (2023-10-26) * [0036], [0037], [0048] * ----- | 1-15 | |
| A | EP 4 253 987 A1 (KONINKLIJKE PHILIPS NV [NL]) 4 October 2023 (2023-10-04) * [0003], [0016] * ----- | 1-15 | **TECHNICAL FIELDS SEARCHED (IPC)** G16H |

The present search report has been drawn up for all claims

| Place of search | Date of completion of the search | Examiner |
|---|---|---|
| Munich | 22 May 2024 | Sundqvist, Benjamin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                                 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 15 8895

This annex lists the patent family members relating to the patent documents cited in the above-mentioned European search report.
The members are as contained in the European Patent Office EDP file on
The European Patent Office is in no way liable for these particulars which are merely given for the purpose of information.

22-05-2024

| Patent document cited in search report | | Publication date | Patent family member(s) | | Publication date |
|---|---|---|---|---|---|
| CN 109998487 | A | 12-07-2019 | NONE | | |
| US 2023248268 | A1 | 10-08-2023 | NONE | | |
| WO 2022194666 | A1 | 22-09-2022 | CN | 117042697 A | 10-11-2023 |
| | | | EP | 4059433 A1 | 21-09-2022 |
| | | | EP | 4308002 A1 | 24-01-2024 |
| | | | JP | 2024512393 A | 19-03-2024 |
| | | | US | 2024065663 A1 | 29-02-2024 |
| | | | WO | 2022194666 A1 | 22-09-2022 |
| US 2023342914 | A1 | 26-10-2023 | JP | 2023160813 A | 02-11-2023 |
| | | | US | 2023342914 A1 | 26-10-2023 |
| EP 4253987 | A1 | 04-10-2023 | NONE | | |

EPO FORM P0459

For more details about this annex : see Official Journal of the European Patent Office, No. 12/82